# EUROPÄISCHE PATENTSCHRIFT

(11) **EP 0 853 241 B1**
(45) Veröffentlichungstag und Bekanntmachung des Hinweises auf die Patenterteilung: **08.03.2006**
(21) Anmeldenummer: 97250385.8
(22) Anmeldetag: 30.12.1997
(51) Int. Cl.: G01N 33/18

(54) **Verfahren und Vorrichtung zur Grundwassertestung**
Method and apparatus for testing groundwater
Méthode et appareil pour tester l'eau souterraine

(30) Priorität: 06.01.1997 DE 19700872
(43) Veröffentlichungstag der Anmeldung: 15.07.1998
(73) Patentinhaber: UFZ-UMWELTFORSCHUNGSZENTRUM Leipzig-Halle GmbH, 04318 Leipzig (DE)
(72) Erfinder: Müller, Roland A., Dr., 04420 Markranstädt OT Frankenheim (DE); Münker, Thomas, Dr., 53547 Kasbach-Ohlenberg (DE); Bernhard, Katy, 04463 Grosspösna (DE)
(74) Vertreter: Hengelhaupt, Jürgen

(56) Entgegenhaltungen:
- EP-A- 0 261 516
- DE-U- 29 607 630
- US-A- 5 169 532
- US-A- 5 209 851
- US-A- 5 366 634

## Beschreibung

Die Erfindung betrifft ein Verfahren und eine Vorrichtung zur Grundwassertestung und ist anwendbar im Rahmen der Umwelt-Grundwassersanierung und dabei insbesondere für die Durchführung von Maßstabsvergrößerungen labortechnischer Ergebnisse in den halbtechnischen - und Pilotmaßstab.

Es ist bekannt, Grundwassersanierungen auf physikalisch-chemischer oder mikrobiologischer Basis durchzuführen. Vorraussetzung für die Durchführung derartiger Sanierungen sind Untersuchungen im labortechnischen Maßstab, um die Sanierungsparameter festzulegen. Ausgehend hiervon erfolgen Maßstabsvergrößerungen aus dem labortechnischen in den halbtechnischen und in den Pilotmaßstab. Vor allem mikrobiologische in-Situ-Sanierungen von Grundwässern erfordern vor der Planung und Auslegung des großtechnischen Maßstabes umfangreiche Voruntersuchungen.

Hierbei wird üblicherweise zunächst im Labormaßstab der Nachweis der biologischen Abbaubarkeit der jeweiligen Kontamination erbracht. Dies umfaßt insbesondere den Nachweis einer Schadstoff-verwertenden Mikroflora sowie die Bewertung des mikrobiellen Selbstreinigungspotentials. Als erste Maßstabsvergrößerung werden dann Proben des Aquifers in Säulen eingebaut und mit durch die Probenahme gestörtem Grundwasser sowie weiteren Zuschlagstoffen beaufschlagt. Zweckmäßigerweise werden verschiedene verfahrensmöglichkeiten und Verfahrenskombinationen getestet. Aus den Ergebnissen dieser halbtechnischen Versuche werden Kosten und Dauer einer Sanierungsmaßnahme sowie die Sanierungseffizienz abgeschätzt, es erfolgt also eine Überprüfung auf technische Realisierung labortechnischer Erkenntnisse.

Diese Untersuchungen gemäß dem bekannten Stand der Technik finden im allgemeinen im Labor bzw. im Technikum statt. Hier stellt sich jedoch das prinzipielle Problem, daß das benötigte Grundwasser durch den Beprobungsvorgang und den sich anschließenden Transport in seiner Zusammensetzung chemisch verändert wird. Grundwässer liegen üblicherweise unter anoxischen/anaeroben Bedingungen vor. Die Grundwasserinhaltsstoffe befinden sich daher im reduzierten Zustand. Eine Beprobung, ein Umpumpen, ein Transport und/oder eine Zwischenlagerung stellen somit einen massiven Eingriff in die in-Situ-Parameter dar. Eine Einschätzung der technischen, finanziellen und zeitlichen Sanierungsparameter der halbtechnischen Phase, also der technischen Realisierung der Ergebnisse der Laborphase, ist somit nur mit erheblichen Einschränkungen möglich.

Der Erfindung liegt daher die Aufgabe zugrunde, ein Verfahren und eine Vorrichtung zu schaffen, welche mit einfachen Mitteln und universell einsetzbar die effektive Ermittlung der Sanierungsparameter gestatten sowie eine flexible und rentable Nutzung der Vorrichtungskomponenten zu ermöglichen.

Diese Aufgabe wird erfindungsgemäß gelöst durch die Merkmale im kennzeichnenden Teil der Ansprüche 1 und 12 in Verbindung mit den Merkmalen im jeweiligen Oberbegriff.
Zweckmäßige Ausgestaltungen der Erfindung sind in den Unteransprüchen enthalten.

Ein besonderer Vorteil der Erfindung besteht darin, daß zur Ermittlung und Optimierung der Sanierungsparameter für die großtechnische Sanierung ein hocheffizientes Vorgehen bei der Verfahrensentwicklung ermöglicht wird, indem im Grundwassersanierungsgebiet oder in dessen unmittelbarer Nähe mobile Testeinheiten für Untersuchungen im klein-/halbtechnischen Maßstab und/oder Untersuchungen im Pilotmaßstab positioniert werden, die mobilen Testeinheiten mit mindestens einem Grundwasserstrom direkt verbunden werden und den mobilen Testeinheiten das kontaminierte Grundwasser aus mindestens einem Förderstrom zugeführt wird, dem zugeführten Grundwasser Nährlösungen für Mikroorganismen und/oder Zuschlagstoffe zudosiert werden, das so vorbehandelte Grundwasser Festbettreaktoren oder Säuleneinheiten zugeleitet wird, von dem Grundwasser vor und nach Verlassen der Festbettreaktoren oder der Säuleneinheiten Sauerstoffgehalt und/oder Redoxpotential und/oder pH-Wert gemessen werden, und das analysierte Grundwasser abschließend reinfiltriert oder abgeleitet wird.

Ein weiterer Vorteil der Erfindung besteht in der universellen Einsetzbarkeit und hohen Mobilität und Flexibilität der Vorrichtungskomponenten, wobei im Grundwassersanierungsgebiet oder in dessen unmittelbarer Nähe mindestens eine mobile Testeinheit für Untersuchungen im klein-/halbtechnischen Maßstab GW 1 und/oder mindestens eine mobile Testeinheit für Untersuchungen im Pilotmaßstab GW 2 angeordnet ist und in der mobilen Testeinheit GW 1 mit dem Grundwasserstrom über mindestens einen Förderstrom direkt verbundene Säuleneinheiten angeordnet sind, wobei die Säuleneinheiten mit einem Festbett gefüllte Säulen S aufweisen, welche parallel oder in Reihe geschaltet sind und in der mobilen Testeinheit GW 2 für Untersuchungen im Pilotmaßstab der originale Bodenkörper als Festbettreaktor dient und zusätzlich zu den mobilen Testeinheiten GW 1 und GW 2 mindestens eine mobile Laboreinheit GW 3 angeordnet ist.

Die Erfindung soll nachstehend anhand von zumindest teilweise in den Figuren dargestellten Ausführungsbeispielen näher erläutert werden. Es zeigen:
- Fig. 1: eine Übersichtsdarstellung einer möglichen Anordnung zweier mobiler Testeinheiten und einer mobilen Laboreinheit
- Fig. 2: ein Fließbild mit den wesentlichen Komponenten einer Containerausstattung für Untersuchungen im klein-/halbtechnischen Maßstab
- Fig. 3: eine Säuleneinheit mit einer Säule in Modulbauweise
- Fig. 4: ein Fließbild mit den wesentlichen Komponenten einer Containerausstattung für Untersuchungen im Pilotmaßstab
- Fig. 5: eine mögliche Ausstattung der Laboreinheit

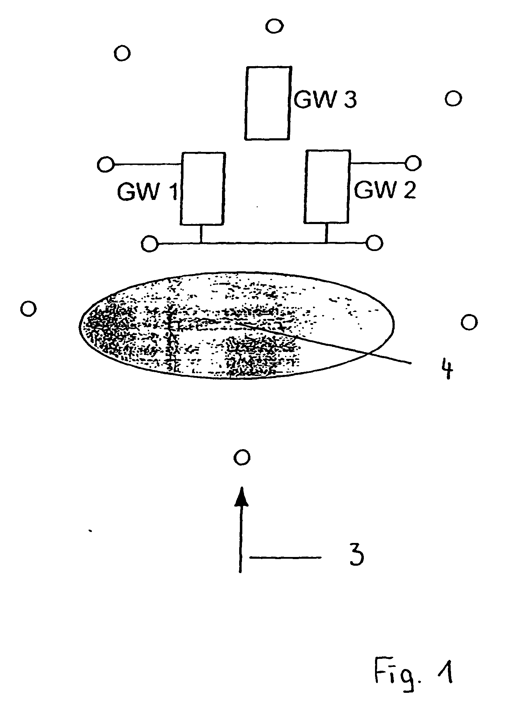

Wie aus Fig. 1 zu ersehen ist, besteht die mobile Testkonfiguration aus drei Komponenten, die wie folgt eingesetzt werden:
a) Container mit Testeinheit für Untersuchungen im klein-/halbtechnischen Maßstab GW 1
b) Container mit Testeinheit für Untersuchungen im Pilotmaßstab GW 2
c) Container mit Laboreinheit für mikrobiologische und physiko-chemische Untersuchung während der Versuche GW 3

Jede Komponente ist in einem Euronorm-Container untergebracht. Die Container sind kranbar und im öffentlichen Straßenverkehr ohne besondere Auflagen transportierbar. Sie können über Stecker bzw. Anschlußverbindungen mit Strom und/oder Wasser und/oder Gas versorgt werden. Jede Komponente ist so konzipiert, daß sie einzeln voll funktionsfähig ist.

Das Realisierungskonzept ermöglicht eine Verfahrensentwicklung unter Beibehaltung und Gewährleistung der in-Situ-Parameter bei einer gleichzeitig universell einsetzbaren Grundausstattung. Ein wesentlicher Bestandteil des Konzeptes ist die unmittelbare Standortnähe der Versuchscontainer zum kontaminierten Grundwasser. Die einzelnen Komponenten GW 1 und GW 2 werden direkt in den Förderstrom eingebunden. Eine Störung des Grundwassers durch Beprobung und Transport wird dadurch verhindert. Das gereinigte bzw. analysierte Grundwasser kann infiltriert oder in einen Vorfluter bzw. in die Kanalisation geleitet werden.

Die Containerkonfiguration erlaubt eine flexible Nutzung der Anlagenbestandteile. Die Anlagencontainer sind autark nutzbar, sie können sowohl als Einzelcontainer, gemeinsam oder in geeigneten Kombinationen wie beispielsweise GW 1 und GW 3 oder GW 2 und GW 3 eingesetzt werden.

Die Anlagenkonfiguration ist für einen universellen Einsatz ausgelegt und kann für halbtechnische Untersuchungen zur Erprobung von "pump & treat" Varianten, für subterrestische Sanierungsvarianten sowie Varianten der passiven Grundwasserbehandlung, z.B. "funnel & gate"-Systeme eingesetzt werden. Aufgrund der Flexibilität der Anlagenkonfiguration können neben mikrobiologischen Sanierungsvarianten auch physiko-chemische Verfahren angewendet, entwickelt oder überprüft werden. Im Rahmen mikrobiologischer Fragestellungen können mehrere prinzipielle Sanierungsvarianten (unterschiedliche Elektronenakzeptoren für die Mikroorganismen) eingesetzt werden:
- aerobe Sanierungsvarianten (z.B. H₂O₂-Dosierung für den Kohlenwasserstoffabbau)
- anerobe oder anoxische Verfahren (z.B. zum Abbau von Benzol, Toluol, Ethylbenzol und Xylol unter denitrifizierenden Bedingungen mit Nitrat als terminalen Elektronenakzeptor)
- aerobe/anaerobe Kombinationsverfahren (z.B. zum Abbau von hochchlorierten Kohlenwasserstoffen)

Die mobile Testeinheit für Untersuchungen im klein-/halbtechnischen Maßstab dient als Entwicklungsplattform für Sanierungsvarianten der Grundwasserbehandlung. Hierfür werden sechs Festbettreaktoren, im folgenden Säuleneinheiten genannt, vom Grundwasser durchströmt. Die einzelnen Säuleneinheiten können wahlweise mit einem von zwei verschiedenen Grundwasserströmen beschickt werden. Das Grundwasser gelangt aus Förderbrunnen zu der mobilen Testeinheit GW 1 im Container, um dort in einen Grundwasserstrom eingespeist zu werden. Es besteht die Möglichkeit, zwei voneinander unabhängige Grundwasserströme (GWS 1 und GWS 2) zu nutzen.

Als Festbett können Aquifere (ungestört oder gestört entnommen), Bodenmaterial sowie konventionelle Trägermaterialien zum Einsatz kommen. Eine weitere Anwendungsmöglichkeit liegt in der Erprobung von Füllmaterialien für "funnel & gate"-Systeme. So können im vorliegenden Ausführungsbeispiel sechs verschiedene "gate"-Materialien gleichzeitig bei ansonsten gleichen Versuchsbedingungen vergleichend überprüft werden. Desweiteren besteht die Möglichkeit, bei gleichbleibendem Material die Prozeßführung durch das Einstellen verschiedener Verweilzeiten sowie durch unterschiedliche physikalisch-chemische Bedingungen zu optimieren. Ein derartiges Vorgehen kann rechtzeitig den Einsatz nicht geeigneter Materialien in "gate"-Systeme verhindern und somit neben einer erheblichen Kosteneinsparung auch zur Gewährleistung der Betriebssicherheit beitragen.

Aufgabe der halbtechnischen Phase der Verfahrensentwicklung ist die Überprüfung der technischen Realisierung der Erkenntnisse des Labormaßstabes. Eine realistische Abschätzung von Sanierungsdauer, Sanierungszielen und Sanierungskosten wird hierdurch möglich.

Mit der mobilen Testeinheit GW 2 sollen in-situ-Pilotversuche realisiert werden, wobei der Bodenkörper als Festbettreaktor dient, aber auch biologische "gate"-Systeme mit Zuschlagstoffen beaufschlagt werden können. Die Ergebnisse dieser Versuche können direkt zur Maßstabsvergrößerung (Sanierungsmaßstab) verwendet werden. Eine Konkretisierung der Aussagen zu Sanierungsdauer, Sanierungszielen und Sanierungskosten wird durch die Anwendung des GW 2 möglich.

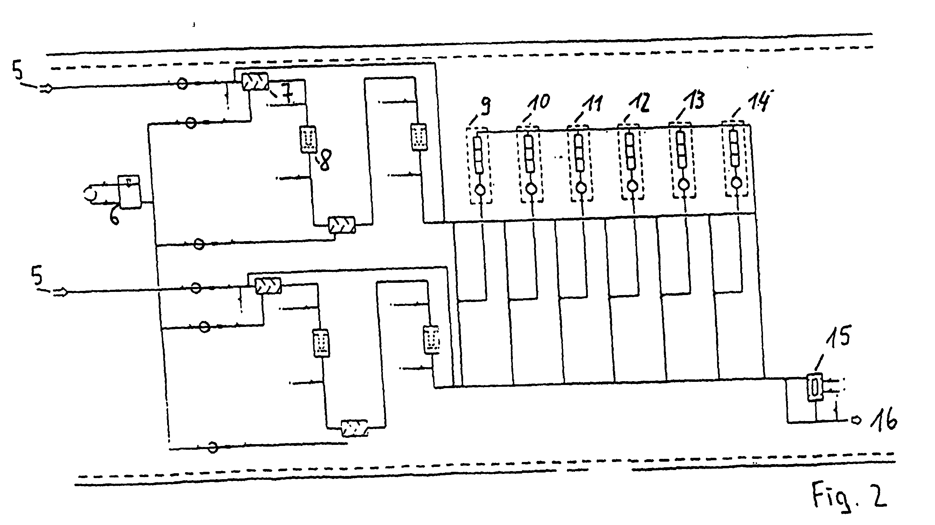

Nachfolgend soll der Aufbau der mobilen Testeinheiten beschrieben werden. Die in einem mobilen Euro-Container angeordnete Testeinheit GW 1, wie in Fig. 2 dargestellt, besteht aus folgenden Komponenten:
- Grundwasserentnahme
- Vorbehandlung, beispielsweise Enteisenung, Entmanganung, auch über Bypass zu umgehen
- Dosierung von Nähr- und Zuschlagstoffen
- sechs Säuleneinheiten als Festbettreaktoren in Modulbauweise

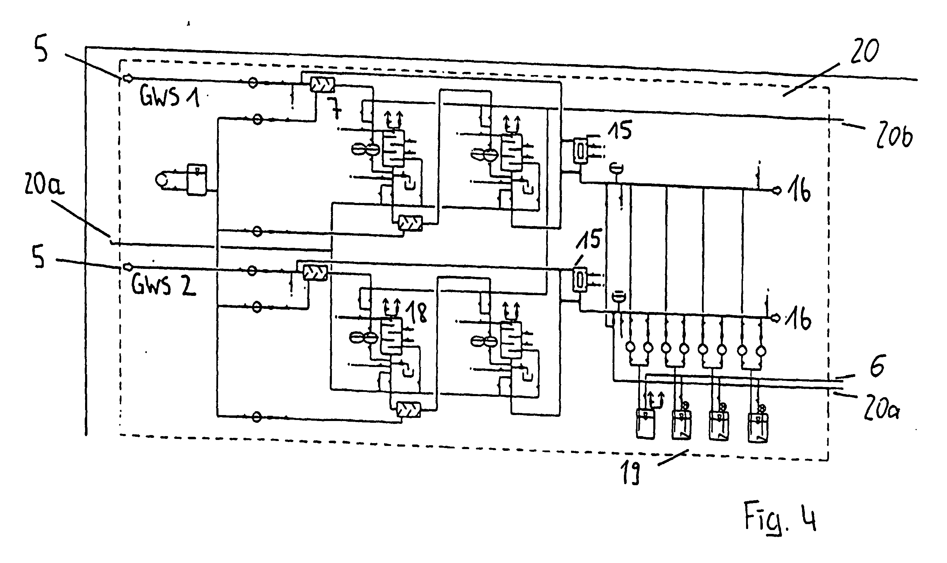

Die mobile Testeinheit GW 2, wie in Fig. 4 dargestellt, besteht aus folgenden Komponenten:
- Grundwasserentnahme
- Vorbehandlung, beispielsweise Enteisenung, Entmanganung, auch über Bypass zu umgehen
- Naßaktivkohlefilter, auch über Bypass zu umgehen
- Dosierung von Nähr- und Zuschlagstoffen
- natürlichen Bodenkörper als Festbettreaktor

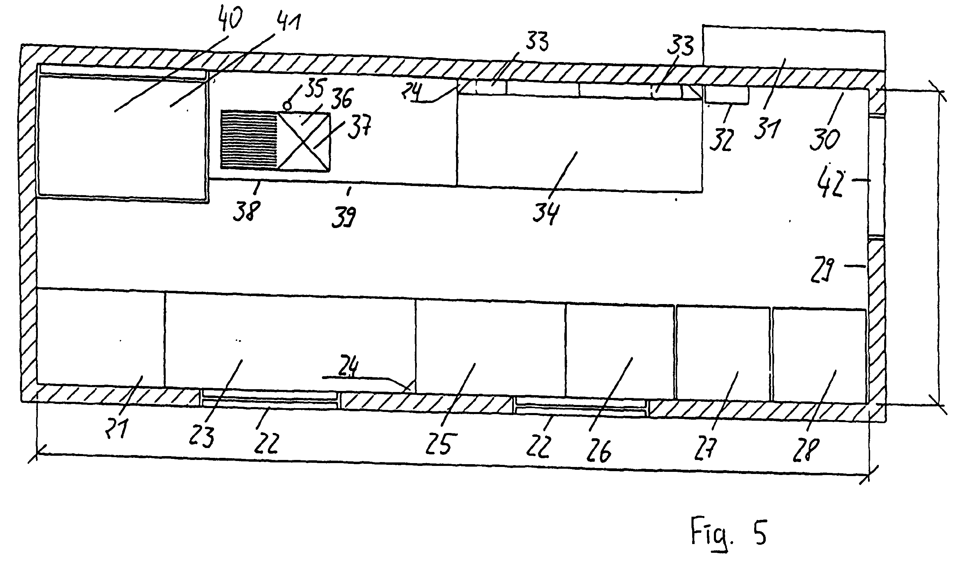

In der mobilen Laboreinheit (Fig. 5) befinden sich als wesentliche Ausrüstungskomponenten:
- einige Laborarbeitstische
- ein Abzugschrank
- Schreibarbeitsplätze
- Kühl- und Gefrierschrank

Der dynamische Ablauf in der mobilen Testeinheit GW 1 vollzieht sich wie folgt:
Im Container befinden sich im vorliegenden Ausführungsbeispiel insgesamt sechs Säulen S mit einem Volumen von je ca. 26 l, welche mittels Doppelmantel temperiert werden können. Die Säulen S können sowohl nacheinander als auch parallel betrieben werden. Wegen der Notwendigkeit einer optischen Kontrolle, beispielsweise der Entstehung von aeroben oder anaeroben Zonen oder bevorzugten Fließzonen, sind die Säulen S aus Glas gefertigt. Seitliche Probenahmestellen P ermöglichen die Entnahme von Grundwasserproben.
Die Versorgung der Säulen S mit Grundwasser kann im vorliegenden Ausführungsbeispiel wahlweise aus zwei verschiedenen Förderbrunnen, Grundwasserstrom GWS 1 bzw. GWS 2 erfolgen.

Für Sanierungsvarianten, für die eine Vorbehandlung des Grundwassers erforderlich ist, kann als erste Verfahrensstufe eine Enteisenung und Entmanganung (Fällung und Filtration) zugeschaltet werden. Die Beaufschlagung der Säuleneinheiten mit dem Grundwasser erfolgt jeweils über Vorlagepumpen. Damit kann der Durchsatz für jede Säule S individuell eingestellt werden (Simulation einer Langsam- oder Schnellinfiltration des Aquifers). Das benötigte Grundwasser wird mit einem konstanten Volumenstrom aus den Grundwasserströmen GWS 1 und GWS 2 entnommen. In die Zuleitungen zu den Säulen können drei verschiedene Nährlösungen NL und Zuschlagstoffe dosiert werden.
Beispiele für derartige Nährlösungen sind:
- Ammoniumsulfat
- Dikaliumhydrogenphosphat
- Vitaminlösungen.

Beispiele für derartige Zuschlagstoffe sind:
- Wasserstoffperoxid
- Nitrat
- Co-Substrate (z.B. Methan).

Nach der Dosierung der Nährlösungen und/oder der Zuschlagstoffe erfolgt die Messung der Parameter Volumenstrom und/oder Druck und/oder Redoxpotential und/oder pH-Wert und/oder Sauerstoffkonzentration.

Im Abstrom der Säule S werden die Parameter Redoxpotential und/oder Sauerstoffkonzentration und/oder pH-Wert gemessen. Die Meßwerte werden über ein Meßwerterfassungs- und Speichersystem aufgenommen, gespeichert und on-line dargestellt.

Überschüssiges Wasser aus den Grundwasserströmen GWS 1 bzw. GWS 2 sowie dem Ablauf der Säulen S wird vor der Einleitung oder Reinfiltration über einen Naßaktivkohlefilter gereinigt. Auch hier ist wahlweise über einen Bypass eine Direktinfiltration möglich. Im und hinter dem Naßaktivkohlefilter können Wasserproben entnommen werden, um rechtzeitig einen Schadstoffdurchbruch feststellen zu können.

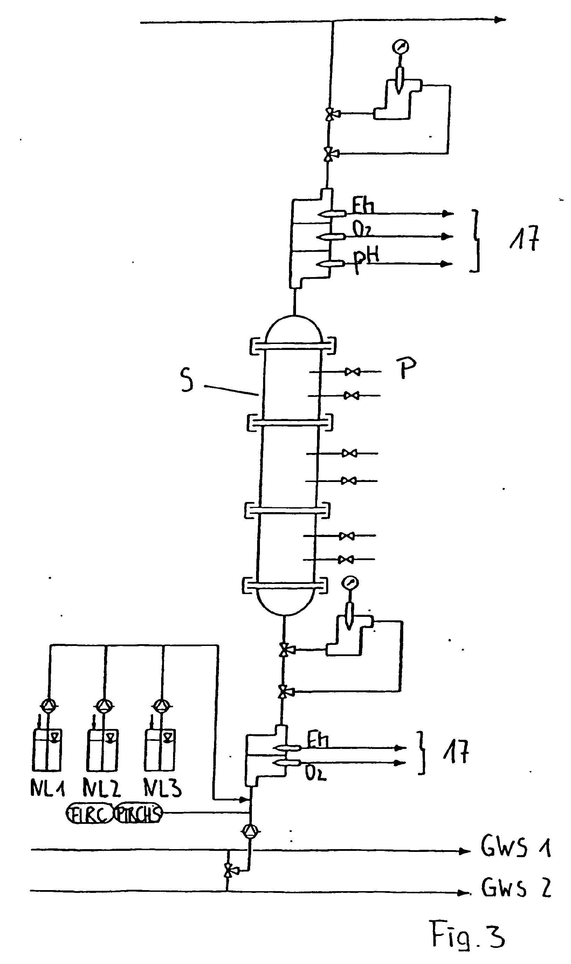

Eine Säuleneinheit ist in Fig. 3 dargestellt und besteht im wesentlichen aus den Vorlagen für Nähr- und Zuschlagstoffe mit Dosiereinrichtung, einer Vorlagepumpe, der Säule S in Modulbauweise mit Probenahmestutzen und Meß- und Regeltechnik.

Die Säule S ist aus Glas gefertigt und besteht aus den fünf Doppelmantelsegmenten unterer Abschluß, Fußsegment, Mittelsegment, Kopfsegment und oberer Abschluß. Dieser Aufbau erlaubt eine variable Säulenlänge sowie ein einfaches Befüllen, Entleeren und Reinigen der Säule S. Insgesamt sind bei dem vorliegenden Ausführungsbeispiel sechs seitliche Entnahmestellen P für die Beprobung des Grundwassers angeordnet.

Das Festbett der Säulen S besteht je nach beabsichtigter Anwendung aus Aquifer (gestört oder ungestört entnommen), Boden, alternativen Trägermaterialien sowie "gate"-Materialien (wie z.B. Fe°).

Abschließend sollen zwei Einsatzbeispiele beschrieben werden.

### I. Biologische Behandlung

Untersuchungen zur "End of Pipe"-Technologie, Voruntersuchungen zur subterrestischen Behandlung sowie ein biologisch aktiviertes "Gate".

Der Grundwasserzustrom in die Säuleneinheit erfolgt z.B. über Schlauchpumpen. Durch ein Drei-Wege-Ventil kann zwischen den beiden Grundwasserströmen GWS 1 und GWS 2 ausgewählt werden. Eine Optimierung der Verweilzeit kann über unterschiedliche Volumenströme realisiert werden. In den Zustrom (Langsam- oder Schnellinfiltration) werden die entsprechenden Nähr- und Zuschlagstoffe dosiert. Danach erfolgt die Messung des Sauerstoffgehaltes, des Redoxpotentials und des pH-Wertes. Das so vorbereitete Grundwasser durchströmt das Festbett. Durch die Optimierung der mikrobiellen Milieubedingungen werden die autochtonen oder zu inokulierenden Mikroorganismen zum Schadstoffabbau angeregt. Im Abstrom der Säule werden Sauerstoffgehalt, Redoxpotential und pH-Wert gemessen. Um rechtzeitig ein Zusetzen der Festbettporen bzw. Festbettzwischenräume durch Verockerung, Biomassebewuchs oder Sauerstoffblasen erkennen zu können, wird vor und nach der Säule der Druck gemessen. Übersteigt die Druckdifferenz einen bestimmten Wert, werden sämtliche Pumpen abgeschaltet, um ein Bersten der Säule zu verhindern.

### II. Physiko-chemische Behandlung

Der Zustrom zur Säule erfolgt mittels Schlauchpumpen. Durch ein Drei-Wege-Ventil kann zwischen den beiden Grundwasserströmen GWS 1 und GWS 2 ausgewählt werden. Eine Optimierung der Verweilzeit kann über unterschiedliche Volumenströme realisiert werden. Das Grundwasser durchströmt das Festbett. Im Abstrom der Säule werden Sauerstoffgehalt, Redoxpotential und pH-Wert gemessen. Um rechtzeitig eine Verstopfung der Säule durch Verockerung, Biomassebewuchs oder Sauerstoffblasen erkennen zu können, wird vor und nach der Säule Druck gemessen. Übersteigt die Druckdifferenz einen bestimmten Wert, werden sämtliche Pumpen abgeschaltet, um ein Bersten der Säule zu verhindern.

Die Erfindung ist nicht beschränkt auf die hier dargestellten Ausführungsbeispiele. Vielmehr ist es möglich, durch Kombination der genannten Mittel und Merkmale weitere Ausführungsvarianten zu realisieren, ohne den Rahmen der Erfindung zu verlassen.

### Bezugszeichenliste

- 1: Schluckbrunnen
- 2: Förderbrunnen
- 3: Grundwasserfließrichtung
- 4: Kontamination
- 5: Zulauf von der Übergabestelle
- 6: Zuschlagstoff H₂O₂
- 7: Rohmischer
- 8: Kerzenfilter
- 9: Säuleneinheit S1
- 10: Säuleneinheit S2
- 11: Säuleneinheit S3
- 12: Säuleneinheit S4
- 13: Säuleneinheit S5
- 14: Säuleneinheit S6
- 15: Aktivkohle
- 16: Ablauf zur Übergabestelle
- 17: Meßwerterfassungs- und Speichersystem
- 18: Mehrschichtfilter
- 19: Vorlagebehälter
- 20: Liefergrenze
- 20a: von der Stadtwasserleitung
- 20b: zum Spülwasserauffangbehälter
- 21: Hängeschrank
- 22: Fenster
- 23: Arbeitstische mit Unterschränke (abschließbar)
- 24: Versorgungsleiste
- 25: Schreibtisch mit Unterschrank (abschließbar)
- 26: EDV-Arbeitstisch
- 27: Kühlschrank/ Hängeschrank
- 28: Gefrierschrank/ Hängeschrank
- 29: Notbrause
- 30: Feuerlöscher
- 31: Gasflaschen
- 32: Erste Hilfe Schrank
- 33: Ablauf
- 34: Arbeitstisch mit Ablauf und Unterschränken
- 35: Mischbatterie
- 36: Spüle
- 37: Heißwassergerät
- 38: Spülunterbau
- 39: Spüll-Gerät unter Tisch
- 40: Abzugsschrank Typ AZ 12
- 41: Ventilator über Dach
- 42: Tür

## Patentansprüche

1. Verfahren zur Grundwassertestung im klein-/halbtechnischen - und Pilotmaßstab zur Ermittlung und Optimierung der Sanierungsparameter, wobei
- im Grundwassersanierungsgebiet oder in dessen unmittelbarer Nähe mobile Testeinheiten für Untersuchungen im klein-/halbtechnischen Maßstab und/oder Untersuchungen im Pilotmaßstab positioniert werden,
- die mobilen Testeinheiten mit mindestens einem Grundwasserstrom direkt verbunden werden und somit die in-situ-Parameter erhalten bleiben,
- den mobilen Testeinheiten das kontaminierte Grundwasser aus mindestens einem Förderstrom zugeführt wird,
- dem zugeführten Grundwasser Nährlösungen für Mikroorganismen und/oder Zuschlagstoffe zudosiert werden,
- das so vorbehandelte Grundwasser Festbettreaktoren oder Säuleneinheiten zugeleitet wird,
- von dem Grundwasser vor und nach Verlassen der Festbettreaktoren oder Säuleneinheiten Sauerstoffgehalt und/oder Redoxpotential und/oder pH-Wert gemessen werden,
- und das analysierte Grundwasser abschließend reinfiltriert oder abgeleitet wird.

2. Verfahren nach Anspruch 1,
**dadurch gekennzeichnet, daß**
zusätzlich zu den mobilen Testeinheiten mindestens eine mobile Laboreinheit positioniert wird.

3. Verfahren nach Anspruch 1,
**dadurch gekennzeichnet, daß**
in der Grundwasserzuleitung vor den Säuleneinheiten und in der Grundwasserableitung hinter den Säuleneinheiten der Druck gemessen und die Druckdifferenz ausgewertet wird.

4. Verfahren nach Anspruch 3,
**dadurch gekennzeichnet, daß**
bei Übersteigen einer festgelegten Druckdifferenz eine Havarieabschaltung erfolgt.

5. Verfahren nach Anspruch 1,
**dadurch gekennzeichnet, daß**
die gemessenen Werte Sauerstoffgehalt und/oder Redoxpotential und/oder pH-Wert erfaßt, gespeichert und weiterverarbeitet werden.

6. Verfahren nach Anspruch 1,
**dadurch gekennzeichnet, daß**
die mobilen Testeinheiten einzeln, gemeinsam oder untereinander kombiniert eingesetzt werden.

7. Verfahren nach Anspruch 1,
**dadurch gekennzeichnet, daß**
die Nährlösungen Ammoniumsulfat und/oder Dikaliumhydrogenphosphat und/oder Vitaminlösungen dosiert werden.

8. Verfahren nach Anspruch 1,
**dadurch gekennzeichnet, daß**
die Zuschlagstoffe Wasserstoffperoxid und/oder Nitrat und/oder Co-Substrate wie z.B. Methan dosiert werden.

9. Verfahren nach Anspruch 1,
**dadurch gekennzeichnet, daß**
das den mobilen Testeinheiten zugeführte Grundwasser enteisent und entmangant wird.

10. Verfahren nach Anspruch 1,
**dadurch gekennzeichnet, daß**
das analysierte Grundwasser vor der Ableitung oder Reinfiltration über Naßaktivkohlefilter gereinigt wird.

11. Verfahren nach Anspruch 1,
**dadurch gekennzeichnet, daß**
der Grundwasserzustrom in die Säuleneinheiten über Schlauchpumpen realisiert wird.

12. Mobile Testvorrichtung zur Grundwassertestung im Grundwassersanierungsgebiet oder in dessen unmittelbarer Nähe im klein-/halbtechnischen - und Pilotmaßstab zur Ermittlung und Optimierung der in-situ Sanierungsparameter,
**gekennzeichnet durch**
- mindestens einen Container mit einer Testeinheit für Untersuchungen im klein-/halbtechnischen Maßstab (GW 1), in der Säuleneinheiten angeordnet sind, die mit einem Festbett gefüllte Säulen (S) aufweisen, welche parallel oder in Reihe geschaltet sind, und die mit dem Grundwasserstrom über mindestens einen Förderstrom direkt verbunden sind, und/oder
- mindestens einen Container mit einer Testeinheit für Untersuchungen im Pilotmaßstab (GW 2), wobei der Bodenkörper als Festbettreaktor dient.

13. Vorrichtung nach Anspruch 12,
**dadurch gekennzeichnet, daß**
zusätzlich zu den mobilen Testeinheiten (GW 1, GW 2) mindestens eine mobile Laboreinheit (GW 3) angeordnet ist.

14. Vorrichtung nach Anspruch 12 oder 13,
**dadurch gekennzeichnet, daß**
die mobilen Testeinheiten (GW 1, GW 2) und die mobile Laboreinheit (GW 3) in Containern angeordnet sind.

15. Vorrichtung nach Anspruch 14,
**dadurch gekennzeichnet, daß**
die Container kranbare Euronormcontainer sind und Anschlüsse für Versorgungsmedien wie Strom und/oder Wasser und/oder Gas aufweisen.

16. Vorrichtung nach Anspruch 12,
**dadurch gekennzeichnet, daß**
in der mobilen Testeinheit (GW 1) insgesamt sechs Säulen (S1-S6) angeordnet sind.

17. Vorrichtung nach Anspruch 12,
**dadurch gekennzeichnet, daß**
die Säulen (S) mit Aquifere und/oder Bodenmaterial und/oder Trägermaterial und/oder "gate"-Materialien gefüllt sind.

18. Vorrichtung nach Anspruch 12,
**dadurch gekennzeichnet, daß**
die Säulen (S) aus Glas bestehen und einen Doppelmantel zur Temperierung aufweisen.

19. Vorrichtung nach Anspruch 18,
**dadurch gekennzeichnet, daß**
die Säulen aus mehreren Segmenten bestehen und seitliche Entnahmestellen (P) für die Beprobung aufweisen.

20. Vorrichtung nach Anspruch 19,
**dadurch gekennzeichnet, daß**
die Säulen (S) die Segmente unterer Abschluß, Fußsegment, Mittelsegment, Kopfsegment und oberer Abschluß aufweisen.

## Claims

1. Method for testing groundwater on a small/semi-industrial and pilot scale to determine and optimise the sanitation parameters, wherein
- in the groundwater sanitation area or in the immediate vicinity thereof, mobile test units for testing on a small/semi-industrial scale and/or testing on a pilot scale are positioned,
- the mobile test units are connected directly with at least one groundwater stream and thus ensure the continuous acquisition of the in situ parameters,
- contaminated groundwater from at least one feed stream is supplied to the mobile test units,
- metered additions of nutrient solutions for micro-organisms and/or additives are made to the supplied groundwater,
- the groundwater pre-treated in this way is supplied to fixed bed reactors or column units,
- the oxygen content and/or redox potential and/or pH value of the groundwater are measured before and after leaving the fixed bed reactors or column units,
- and, finally, the analysed groundwater is re-infiltrated or drained.

2. Method according to claim 1
**characterised in that**
in addition to the mobile test units, at least one laboratory unit is positioned.

3. Method according to claim 1
**characterised in that**
in the groundwater feed pipe in front of the column units and in the groundwater drain behind the column units, the pressure is measured and the pressure differential evaluated.

4. Method according to claim 3
**characterised in that**
in the event of an overshoot of a fixed pressure differential, an emergency shutdown takes place.

5. Method according to claim 1
**characterised in that**
the oxygen content and/or redox potential and/or pH values measures are recorded, stored and processed.

6. Method according to claim 1
**characterised in that**
the mobile test units are positioned individually, together or in combination with each other.

7. Method according to claim 1
**characterised in that**
metered additions of ammonium sulphate and/or dipotassium hydrogen phosphate nutrient solutions and/or vitamin solutions are made.

8. Method according to claim 1
**characterised in that**
metered additions of the additives hydrogen peroxide and/or nitrate and/or co-substrates such as methane are made.

9. Method according to claim 1
**characterised in that**
iron and manganese are extracted from the groundwater supplied to the mobile test units.

10. Method according to claim 1
**characterised in that**
the groundwater analysed is purified via a wet activated carbon filter before being drained or re-infiltrated.

11. Method according to claim 1
**characterised in that**
the groundwater is fed into the column units via hose pumps.

12. Mobile test device for testing groundwater in the groundwater area or in the immediate vicinity thereof on a small/semi-industrial and pilot scale to determine and optimise the in situ sanitation parameters, **characterised in that**
- at least one container with one test unit for testing on a small/semi-industrial scale (GW 1) are arranged in the column units, which comprise columns (S) filled with a fixed bed, which are switched in parallel or in series, and are connected directly to the groundwater stream via at least one feed stream, and/or
- at least one container with one test unit for testing on a pilot scale (GW 2), wherein the sediment bed serves as a fixed bed reactor.

13. Device according to claim 12
**characterised in that**
in addition to the mobile test units (GW 1, GW 2), at least one laboratory unit (GW 3) is arranged.

14. Device according to claim 12 or 13
**characterised in that**
the mobile test units (GW 1, GW 2) and the mobile laboratory unit (GW 3) are arranged in containers.

15. Device according to claim 14
**characterised in that**
the containers are craneable European standard containers and comprise connections for utilities such as power and/or water and/or gas.

16. Device according to claim 12
**characterised in that**
in the mobile test unit (GW 1), a total of six columns (S1-S6) are arranged.

17. Device according to claim 12
**characterised in that**
the columns (S) are filled with aquifers and/or soil material and/or base material and/or gate materials.

18. Device according to claim 12
**characterised in that**
the columns (S) consist of glass and comprise a double casing for temperature equalisation.

19. Device according to claim 18
**characterised in that**
the columns consist of several segments and comprise side removal points (P) for sampling.

20. Device according to claim 16
**characterised in that**
the columns (S) comprise the bottom end, base, central, top and top edge segments.

## Revendications

1. Procédé pour tester l'eau souterraine à l'échelle micro- ou semi-industrielle et à l'échelle pilote pour l'établissement et l'optimisation des paramètres d'assainissement, dans lequel
- des unités de test mobiles pour des analyses à l'échelle micro- ou semi-industrielle et/ou des analyses à l'échelle pilote sont mises en place dans la zone d'assainissement de l'eau souterraine ou à proximité immédiate de celle-ci,
- les unités de test mobiles sont reliées directement à au moins un courant d'eau souterraine de telle sorte que les paramètres in situ sont maintenus,
- l'eau souterraine contaminée issue d'au moins un courant d'adduction est amenée aux unités de test mobiles,
- des bouillons de culture de micro-organismes et/ou des agrégats sont ajoutés par dosage à l'eau souterraine amenée,
- l'eau souterraine ainsi prétraitée est amenée à des réacteurs à lit fixe ou à des unités de colonnes,
- la teneur en oxygène et/ou le potentiel d'oxydoréduction et/ou le pH de l'eau souterraine sont mesurés avant et après que celle-ci soit sortie des réacteurs à lit fixe ou des unités de colonne,
- et l'eau souterraine analysée est ensuite ré-infiltrée ou évacuée.

2. Procédé selon la revendication 1,
**caractérisé en ce que**
au moins une unité de laboratoire mobile est mise en place en complément des unités de test mobiles.

3. Procédé selon la revendication 1,
**caractérisé en ce que**
la pression est mesurée et la différence de pression est évaluée dans l'adduction d'eau souterraine avant les unités de colonne et dans l'évacuation d'eau souterraine après les unités de colonne.

4. Procédé selon la revendication 3,
**caractérisé en ce que**
le dépassement d'une différence de pression déterminée entraîne un arrêt pour avarie.

5. Procédé selon la revendication 1,
**caractérisé en ce que**
les valeurs mesurées de la teneur en oxygène et/ou du potentiel d'oxydoréduction et/ou du pH sont saisies, enregistrées en mémoire et soumises à un traitement supplémentaire.

6. Procédé selon la revendication 1,
**caractérisé en ce que**
les unités de test mobiles sont installées individuellement, conjointement ou en combinaison les unes avec les autres.

7. Procédé selon la revendication 1,
**caractérisé en ce que**
les bouillons de culture sont dosés en sulfate d'ammonium et/ou en phosphate de dicalcium d'hydrogène et/ou en solutions vitaminées.

8. Procédé selon la revendication 1,
**caractérisé en ce que**
les agrégats sont dosés en peroxyde d'hydrogène et/ou en nitrate et/ou en co-substrats tels que le méthane.

9. Procédé selon la revendication 1,
**caractérisé en ce que**
l'eau souterraine amenée aux unités de test mobiles est débarrassée de son fer et de son manganèse.

10. Procédé selon la revendication 1,
**caractérisé en ce que**
l'eau souterraine analysée est purifiée au moyen d'un filtre humide à charbon actif avant d'être évacuée ou ré-infiltrée.

11. Procédé selon la revendication 1,
**caractérisé en ce que**
l'adduction d'eau souterraine dans les unités de colonne est réalisée au moyen de pompes tubulaires.

12. Dispositif de test mobile pour tester l'eau souterraine dans la zone d'assainissement de l'eau souterraine ou à proximité immédiate de celle-ci à l'échelle micro- ou semi-industrielle et à l'échelle pilote pour l'établissement et l'optimisation des paramètres d'assainissement in situ, **caractérisé par**
- au moins un conteneur pourvu d'une unité de test pour des analyses à l'échelle micro- ou semi-industrielle (GW 1), dans laquelle sont agencées des unités de colonnes qui comportent des colonnes (S) remplies d'un lit fixe, connectées en parallèle ou en série, et qui sont reliées directement avec le courant d'eau souterraine par au moins un courant d'adduction, et/ou
- au moins un conteneur pourvu d'une unité de test pour des analyses à l'échelle pilote (GW 2), dans lequel le sédiment sert de réacteur à lit fixe.

13. Dispositif selon la revendication 12,
**caractérisé en ce que**
au moins une unité de laboratoire mobile (GW 3) est mise en place en complément des unités de test mobiles (GW 1, GW 2).

14. Dispositif selon la revendication 12 ou 13,
**caractérisé en ce que**
les unités de test mobiles (GW 1, GW 2) et l'unité de laboratoire mobile (GW 3) sont agencées dans des conteneurs.

15. Dispositif selon la revendication 14,
**caractérisé en ce que**
les conteneurs sont des conteneurs euronormes pouvant être manipulés par grue et comportant des raccords pour supports d'alimentation tels que l'électricité et/ou l'eau et/ou le gaz.

16. Dispositif selon la revendication 12,
**caractérisé en ce que**
six colonnes (S1-S6) au total sont agencées dans l'unité de test mobile (GW 1).

17. Dispositif selon la revendication 12,
**caractérisé en ce que**
les colonnes (S) sont remplies d'aquifères et/ou d'un matériau de sol et/ou d'un matériau support et/ou de matériaux "barrière".

18. Dispositif selon la revendication 12,
**caractérisé en ce que**
les colonnes (S) sont en verre et comprennent un double manteau pour l'équilibrage de la température.

19. Dispositif selon la revendication 18,
**caractérisé en ce que**
les colonnes sont constituées de plusieurs segments et comprennent des points de prélèvement latéraux (P) pour le prélèvement.

20. Dispositif selon la revendication 19,
**caractérisé en ce que**
les colonnes (S) comprennent un segment de fermeture inférieur, un segment de pied, un segment central, un segment de tête et un segment de fermeture supérieur.
